(19) Europäisches Patentamt
European Patent Office
Office européen des brevets

(11) Publication number: **0 315 089 A2**

(12) # EUROPEAN PATENT APPLICATION

(21) Application number: **88118054.1**

(22) Date of filing: **29.10.88**

(51) Int. Cl.4: **C08G 73/06 , C07D 239/22 , C07C 122/00**

(30) Priority: **03.11.87 US 116594**

(43) Date of publication of application: **10.05.89 Bulletin 89/19**

(84) Designated Contracting States: **AT BE CH DE ES FR GB GR IT LI NL SE**

(71) Applicant: **THE DOW CHEMICAL COMPANY**
**2030 Dow Center Abbott Road P.O. Box 1967**
**Midland Michigan 48640-1967(US)**

(72) Inventor: **Wykowski, Paul L.**
**61 Blue Bell Court**
**Lake Jackson Texas 77566(US)**
Inventor: **Puckett, Paul M.**
**126 Daisy**
**Lake Jackson Texas 77566(US)**
Inventor: **Burton, Bruce L.**
**55 Flag Ct.**
**Lake Jackson Texas 77566(US)**

(74) Representative: **Sternagel, Hans-Günther, Dr. et al**
**Patentanwälte Dr. Michael Hann Dr. H.-G. Sternagel Sander Aue 30**
**D-5060 Bergisch Gladbach 2(DE)**

(54) **Curable compositions containing ethylenically unsaturated monomers, polyarylcyanate ester compositions and catalyst.**

(57) Polyarylcyanate compositions are cured by heating at a temperature of from 60 °C to 100 °C in the presence of, as a catalyst, 0.1 to 2 percent by weight of at least one iron, cobalt or zinc salt of a monocarboxylic acid based upon the combined weight of the polyarylcyanate and catalyst.

EP 0 315 089 A2

# CURABLE COMPOSITIONS CONTAINING ETHYLENICALLY UNSATURATED MONOMERS, POLYARYLCYANATE ESTER COMPOSITIONS AND CATALYST

## GOVERNMENT CONTRACT INFORMATION

This invention was made with Government support under contract no. F33615-85-C-5081 awarded by the United States Air Force. The Government has certain rights in this invention.

## FIELD OF THE INVENTION

The present invention relates to curable compositons of polyarylcyanate ester compositions and copolymerizable ethylenically unsaturated monomeric compositions and method for curing the compositions.

## BACKGROUND OF THE INVENTION

Thermoset resins are used in many engineering applications as fiber-reinforced plastics and composites, molded articles, coatings, adhesives and the like. It is desirable that such resins possess temperature and chemical resistance, and are readily processable in molding, extrusion, and hand lay-up processes.

Generally, these polycyanate resins require high temperatures, usually above 120 °C with the usual catalysts to cure to a product having useful properties. Also, such compounds must be either heated to temperatures of 80 °C or more to effectively mix together the catalyst with the resin or use some type of inert diluent to lower the viscosity to effectively process the resin. The first instance limits the ability to mix a resin blend for a low-temperature cure because the viscosity will increase too rapidly to enable a useful article to be made from the blend. In the latter instance, any residual diluent remaining in the cured article will act as a plasticizer and degrade the mechanical and thermal properties of the resin. It would therefore be desirable to have a process and compositions which will cure to a product having useful properties at lower temperatures, e.g. at 100 °C and below.

## SUMMARY OF THE INVENTION

The present invention pertains to a curable composition comprising (A) at least one polyarylcyanate ester composition; (B) at least one ethylenically unsaturated monomer; (C) as a catalyst for curing, at least one iron or zinc complex; and optionally (D) a hydrogen-bonding compound as a curing accelerator; wherein component (A) is present in an amount of from about 50 to about 95 percent by weight based upon the combined weight of components (A) and (B); component (B) is present in an amount of from about 5 to about 50 percent by weight based upon the combined weight of components (A) and (B); component (C) is present in an amount of from about 0.01 to about 2 percent by weight based upon the combined weight of components (A), (B), (C) and (D) and component (D) is present in an amount of from zero to about 10 percent by weight based upon the combined weight of components (A), (B), (C) and (D).

Another aspect of the present invention pertains to an improved method for curing by heating a composition comprising (A) at least one polyarylcyanate ester composition; (B) at least one ethylenically unsaturated monomer; (C) a catalyst and optionally (D) a hydrogen-bonding compound as a curing accelerator; wherein component (A) is present in an amount of from about 50 to about 95 percent by weight based upon the combined weight of components (A) and (B); component (B) is present in an amount of from about 5 to about 50 percent by weight based upon the combined weight of components (A) and (B); component (C) is present in an amount from 0.01 to 2 percent; component (D) is present in an amount of from zero to about 10 percent by weight based upon the combined weight of components (A), (B), (C) and (D); and wherein the improvement resides in employing as the catalyst, at least one selected from the group consisting of iron and zinc complexes in an amount of from about 0.01 to about 2 percent by weight based upon the combined weight of components (A), (B), (C) and (D); and wherein the curing is conducted at a temperature of not greater than 100 °C.

The present invention provides a composition which cures to a product having useful properties when

cured at temperates of 100 ° C and lower.

## DETAILED DESCRIPTION OF THE INVENTION

The curable composition of this invention is a composition which contains polymerization sites and which can be polymerized to form a solid part. The composition comprises a polyarylcyanate ester composition, at least one ethylenically unsaturated monomer, a catalyst therefor and optionally an accelerator for the curing thereof. The composition can contain other comonomers or components which are copolymerizable with the polyarylcyanate ester and/or ethylenically unsaturated monomer. The composition can also contain components which improve the solubility or compatibility of the polyarylcyanate ester composition in the ethylenically unsaturated monomeric composition.

The preferred polyfunctional arylcyanate ester compositions of this invention correspond to the formula:

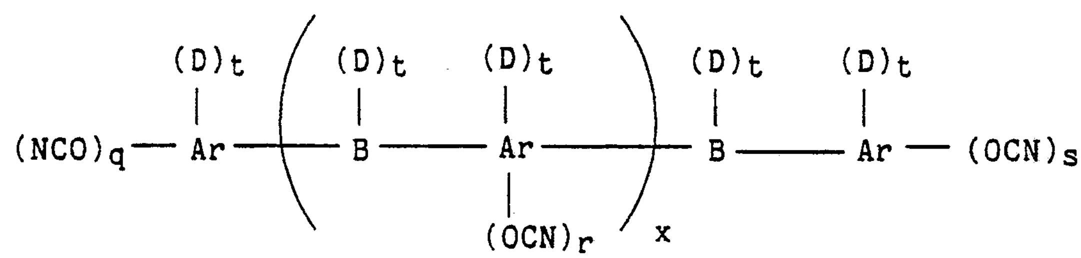

wherein:

Ar is an aromatic moiety;

B is a $C_{1-20}$ acyclic, cyclic or polycyclic aliphatic moiety;

D is independently, in each occurrence, any nonactive hydrogen-containing substituent;

q, r and s are independently, in each occurrence, an integer of 0, 1, 2, or 3; with the proviso that the sum of q, r and s is at least 2;

t is independently, in each oocurrence, an integer of up to 4, inclusive; and

x is a number up to 5, inclusive.

The polyarylcyanate ester composition is a composition which can be polymerized to a cured condition. The composition can be comprised of monomers, oligomers, or a mixture of monomers and oligomers corresponding to the above formula. The mixtures can be referred to as having an average cyanate ester functionality which refers to the average number of cyanate ester groups per molecular unit.

The aromatic moiety is a cyclic carbon-containing moiety which exhibits the (4N + 2) electrons in the n orbital configuration as described in, for example, Morrison & Boyd, Organic Chemistry, 3rd Ed., 1973, page 328. Suitable aryl moieties include cyclic hydrocarbon moieties such as benzene, naphthalene, phenanthrene, anthracene, biaromatic moieties, or 2 or more aromatic moieties bridged by alkylene moieties. Also, heterocyclic moieties such as pyridine are suitable. Preferably, the aromatic moiety is a benzene, naphthalene, biphenyl, binaphthyl, or diphenyl alkylene moiety. Most preferably, the aryl moiety is a benzene moiety.

The arylcyanate ester moieties can be bridged by polycyclic aliphatic bridging moieties. Polycyclic aliphatic bridging moieties provide improved moisture resistance and electrical properties to the polymeric composition compared to aromatic bridging members. Also, the polycyclic aliphatic moieties provide improved mechanical strength and heat resistance to the polymeric composition compared to straight chain aliphatic bridging members.

Polycyclic aliphatic moiety "B" refers herein to an aliphatic moiety which contains two or more cyclic rings. The polycyclic aliphatic moieties can contain one or more double or triple bonds, provided that formation of an aromatic moiety from the cyclic aliphatic moiety is avoided. Examples of suitable polycyclic aliphatic moieties are described in detail in allowed copending application serial no. 828,465, (Atty. docket no. C-33,254) filed February 11, 1986 by Gary W. Bogan and Peter A. Lucus, titled "CURABLE COMPOSITIONS FROM ETHYLENICALLY UNSATURATED MONOMERS AND POLYARYLCYANATE ESTER COMPOSITIONS", all of which is incorporated herein by reference. The preferred polycyclic aliphatic moiety is a moiety which corresponds to one of the formulae II, III, IV, V, VI, VII, VIII or XIII with moieties corresponding to formulae II, III, IV, V or XIII being even more preferred, and moieties corresponding to formula II being most preferred. The formulae referring to those in the aforesaid copending application serial no. 828,465, filed February 11, 1986.

The polyarylcyanate ester compositions of this invention can be in the form of a mixture of many isomers. Further, these polyarylcyanate ester compositions can be a mixture of compounds in which x has a value up to about 5. The value given for x in a particular mixture is an average value.

The most preferred polyarylcyanate ester compositions correspond to the formula

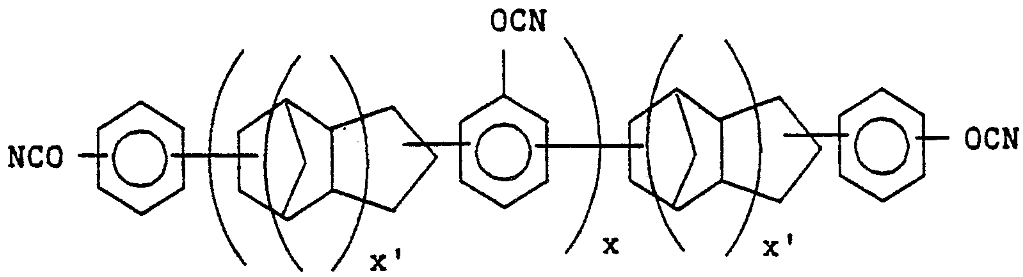

wherein x has an average value from zero up to about 5, inclusive and each x′ independently has an average value from 1 to about 3.

The polyarylcyanate esters which can be employed herein are fully described in the aforementioned copending application serial no. 828,465, filed February 11, 1986.

The polyarylcyanate esters are employed in amounts which correspond to suitably from about 50 to about 95, more suitably from about 70 to about 95, most suitably from about 75 to about 90 percent by weight based upon the combined weight of the polyarylcyanate ester component (A) and the ethylenically unsaturated monomer component (B). The combination of the components then make up 100 percent by weight based upon the combined weights of components (A), (B), (C) and (D).

The ethylenically unsaturated monomeric compositions of this invention comprise at least one ethylenically unsaturated monomer. Suitable monomers include vinyl monomers which contain a vinyl hydrocarbon moiety in a position which is reactive with the arylcyanate ester moieties. Examples of suitable monomers include 1,2-alkenes, ethylenically unsaturated aromatic moieties such as, for example, styrene, divinylbenzene, vinyltoluene; ethylenically unsaturated cyclic hydrocarbons such as cyclopentadiene, dicyclopentadiene and the like; vinylized epoxy resins such as the vinyl ester analogs of the diglycidyl ethers of bisphenol A, such as the Derakane™ vinyl ester resins and the styrene diluted Derakane™vinyl ester resins, acrylic esters, acrylamide monomers, and the like. Other suitable monomers include, allylic aryl esters such as diallylphthalate, allylbenzoate, diallylterephthalate, allylic aryl ethers such as the diallylether of bisphenol A, allyl phenyl ether, diallylether of resorcinol, triallylcyanurate, allylic unsaturated phenolic compounds such as 2-allylphenol, 4,4′-bishydroxy-3,3′-bis(2,3-propenyl)phenyl-2,2-propane, 2,6-diallylphenol, unsaturated mono-, di- and triglycerides (commonly referred to as drying oils), such as dehydrated castor oil, linseed oil, mixtures thereof and the like. Preferably, styrene, toluene, vinyl toluene, and divinylbenzene are employed. The ethylenically unsaturated monomeric compositions of this invention can be substituted with moieties which impart specialized functionality. For example, to impart fire resistance to the cured compositions, the ethylenically unsaturated monomeric composition can be substituted with a suitable halogen such as chlorine, bromine, or fluorine, with bromine being preferred. Examples of suitable fire-retardant monomers are para-bromostyrene, and a reaction product of tetrabromobisphenol A and vinylbenzylchloride; with the reaction product being preferred. The ethylenically unsaturated monomeric compositions of this invention are commerically available, and the methods of making the compositions are well known in the art.

The arylcyanate ester composition is substantially soluble in the ethylenically unsaturated monomeric composition. The arylcyanate ester composition can spontaneously form a thermodynamically stable mixture in the ethylenically unsaturated monomeric composition. Such a mixture can be one in which the molecules of the arylcyanate ester composition is dispersed throughout the molecules of the ethylenically unsaturated monomeric composition as well as forming a micellular or colloidal dispersion and the like.

It is advantageous that the ethylenically unsaturated monomeric composition be in the form of a liquid at between about 15°C and 35°C, preferably about 25°C at atmospheric pressure. However, the monomeric composition can be a solid with a low melting point of between about 35°C and 100°C, preferably between about 40°C and 80°C, and most preferably less than about 70°C. The polyarylcyanate ester composition can be soluble in the melted monomeric composition.

The ethylenically unsaturated monomeric compositions of this invention are employed in the curable composition in a viscosity-reducing amount. Such amount is sufficient to impart a workable viscosity to the curable composition, and/or to provide the desired properties to the cured polymeric composition. Such an

amount can vary, and typically ranges suitably from about 5 up to about 50 weight percent, more suitably up to about 30 weight percent, and most suitably up to about 25 weight percent of the total weight of polyarylcyanate ester component, ethylenically unsaturated monomeric component. The sum of the components make up 100 percent of the composition by weight. It can be desirable to maintain the amount of unsaturated monomer below the stoichiometric amount, because a greater amount will produce vinyl polymerization reaction upon subjecting the curable composition to polymerization conditions. However, such polymerization can be desirable in some circumstances. In view of the fact that the ethylenically unsaturated group will have two sites which can react with an arylcyanate ester moiety, a stoichiometric amount is an amount of about two arylcyanate ester moieties per ethylenically unsaturated moiety on the ethylenically unsaturated monomer.

The stoichiometric amount can be determined by conventional means. For example, the equivalent weight of the polyarylcyanate ester composition can be determined by, for example, gel permeation chromatography. The amount (grams) of polyarylcyanate ester to be polymerized can be divided by the equivalent weight to determine the number of equivalents which are to be polymerized. Since two cyanate moieties can react with one ethylenically unsaturated moiety, the equivalents of polyarylcyanate ester are divided by two to determine the number of equivalents of ethylenically unsaturated monomer to be copolymerized. The number of equivalents of unsaturated monomer can then be multiplied by the molecular weight of the monomer to determine the amount (grams) of unsaturated monomer to be copolymerized with the polyarylcyanate ester composition.

In view of the fact that the preferred polyarylcyanate ester composition is a semi-solid, syrup-like composition, the curable composition of arylcyanate ester composition and ethylenically unsaturated monomeric composition can have a substantially lower solution viscosity than the arylcyanate ester composition alone. The viscosity is dependent upon the amount of unsaturated monomeric composition employed. For example, the viscosity can be lowered by increasing the amount of ethylenically unsaturated monomer which is added to the curable composition. Therefore, the degree of viscosity desired can be controlled by the amount of ethylenically unsaturated monomeric composition added to the curable composition.

The catalyst employed in the curable compositions include the $Fe^{+2}$, $Fe^{+3}$, or $Zn^{+2}$ complexes as described, for example, in Advanced Inorganic Chemistry, 3rd Edition by Cotton & Wilkenson, John Wiley & Sons, 1972, pp 22-23 and 620-629 which is incorporated herein by reference. Suitable complexes include those containing such ligands, for example, as the ionic salts of monocarboxylic acids such as hexanoate, 2-ethylhexanoate, octoate, naphthenate, propionate, oleate, neodecanoate, stearate, tallate, lactate; dicarboxylate anions such as oxalate, malonate succinate, glutarate, adipate, maleate, tartarate, citrate, dithiocarbamates, xanthates; amine compounds such as ethylene diamine, diethylenetriamine, triethylenetetramine; pyridine derivatives such as 2,2′-bipyridine, 1,10-phenanthroline; β-diketonates such as, acetylacetonate, benzolylacetonate, triacetylmethane, 1,1,1-trifluoropentanedione, 2- acetylcyclopentanoate,: phenolic compounds such as phenolate, catecholate, pyrogallolate; phenolic compounds with another electron donating group in the ortho position on the phenyl ring such as salicylic acid, salicylaldehyde, salicylaldimine acylhydrazones of salicylaldehyde; other polydentate ligands such as ethylenediamine tetraacetic acid. Particularly suitable catalysts include, for example, the monocarboxylate acid salts of $Fe^{+2}$, $Fe^{+3}$, and $Zn^{+2}$ having suitably from 1 to about 12, more suitably from 6 to about 12 carbon atoms and β-diketonates. More particularly suitable catalysts include, for example, ferrous octoate, ferric octoate, ferrous naphthenate, ferric naphthenate, ferrous neodecanoate, ferric neodecanoate, ferrous oleate, ferric oleate, ferric acetylacetonate, zinc octoate, zinc naphthenate, zinc 2-ethylhexanoate, zinc neodecanoate, zinc oleate, zinc propionate, zinc acetylacetonate, combinations thereof and the like. These catalysts can be dissolved in a suitable solvent such as mineral spirits, one of the above mentioned reactive monomers, alcohols, ketones, glycol ethers, liquid phenolic compounds, aliphatic esters, aromatic solvents to aid in the dilution of the catalyst into the resin blend. Preferably, the metal carboxylate catalyst can be used in a solid form and mixed directly into the resin blend.

The catalysts are employed in quantities suitably from about 0.01 to about 2, more suitably from about 0.05 to about 1, most suitably from about 0.1 to about 0.75, percent by weight based upon the combined weight of the polyarylcyanate component (A), the ethylenically unsaturated monomer component (B), the catalyst component (C), and, if present, the accelerator component (D).

Suitable accelerators which can be employed, when desired, include, for example, any soluble hydrogen-bonding compound, such as an alcohol, polyol, or a compound containing a phenolic hydroxyl group. Particularly suitable accelerator compounds include, for example, phenol, hydroquinone, 2,6-diallylphenol, 2-allylphenol, 2-hydroxystyrene, ethanol, n-propanol, 2-propanol, higher aliphatic alcohols, ethylene glycol, propylene glycol, benzyl alcohol, glycerol, allylbisphenol A, di-, tri-, and tetraallylbisphenol

A, combinations thereof and the like.

The accelerator compounds are employed suitably in amounts of from zero up to about 10, more suitably from about 0.05 to about 2, most suitably from about 0.5 to about 1.2, percent by weight based upon the combined weight of the polyarylcyanate component, the ethylenically unsaturated monomer component, the catalyst component, and the accelerator component.

The curable composition is cured by heating at a temperature of not greater than 100 °C, suitably at a temperature of from about 25 °C to about 100 °C, more suitably at a temperature of from about 60 °C to about 100 °C. Although the properties of the product resulting from curing at these temperatures are suitable for use, the properties can be improved, if desired, by post curing at tempertures above 100 °C.

The curable compositions can, if desired, contain other materials such as solvents or diluents, fillers, pigments, dyes, flow modifiers, thickeners, reinforcing agents, surfactants, mold release agents, rubber modifiers, coupling agents, combinations thereof and the like.

These additives are added in functional equivalent amounts e.g., the pigments and/or dyes are added in quantities which will provide the composition with the desired color; however, they are suitably employed in amounts of from about zero to about 20, more suitably from about 0.05 to about 5, most suitably from about 0.5 to about 3 percent by weight based upon the weight of the total blended composition.

Solvents or diluents which can be employed herein include, for example, hydrocarbons, ketones, glycol ethers, cyclic ethers, acyclic aliphatic esters, glycol ether esters, alcohols, combinations thereof and the like. Particularly suitable solvents or diluents include, for example, toluene, benzene, xylene, acetone, methyl ethyl ketone, methyl isobutyl ketone, diethylene glycol methyl ether, dipropyl glycol methyl ether, propylene glycol methyl ether, combinations thereof and the like.

The modifiers such as thickeners, flow modifiers and the like can be suitably employed in amounts of from about zero to about 50, more suitably from about zero to about 20, most suitably from about zero to about 10 percent by weight based upon the combined weight of components (A), (B), (C), (D) and the modifier.

The compositions of the present invention can also, if desired, contain reinforcing materials in the form of mats, woven fabric, unidirectional fibers, rovings, random fibers or filaments, inorganic fillers, inorganic whiskers, hollow spheres, ceramics, and the like. These reinforcing materials can be prepared from glass fibers, aramid fibers, graphite fibers, and polymer fibers such as nylon, polyalkylene terephthalate, polyethylene, polypropylene, polyesters, combinations thereof and the like.

The fillers can be employed in amounts suitably from about zero to about 95, more suitably from about 10 to about 80, most suitably from about 40 to about 60 percent by weight based upon the combined weight of total composition.

The following examples are illustrative of the invention, but are not to be construed as to limiting the scope thereof in any manner.

**EXAMPLE 1.**

A hydrocarbon cyanate ester represented by the formula

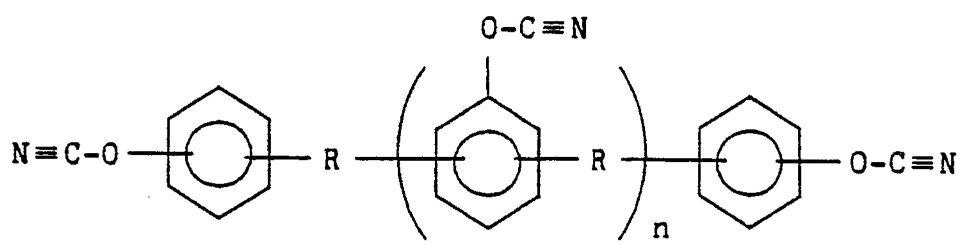

wherein R is a dicyclopentadiene moiety and the average value of n is 1.2 is dissolved in the indicated reactive diluent in the indicated amount.

Into the above solution, enough accelerator, if employed, is added and mixed to provide a solution containing 1 percent by weight of the accelerator. Into this mixture is added and mixed enough metal carboxylate catalyst to provide the indicated metal concentration in the solution (parts per million parts by weight based upon the combined weight of the reactive components). These catalyzed mixtures are poured at room temperature (23 °C) into a test tube and a thermocouple is inserted into the mixture. The test tube is then placed in a hot oil bath at a temperature of 80 °C±0.2 °C. The time interval required for the resin

mixture to go from 65.5 °C to 85.6 °C is defined as the gel time. The time interval between 65.6 °C and the peak exotherm temperature is defined as the cure time. These tests are performed by the SPI Gel/Cure Test method. The results are given in Table I.

The following reactive diluents are employed and all percentages are by weight of resin plus diluent(s).

1. 20% vinyl toluene.
2. 11% vinyl toluene + 4% divinylbenzene.
3. 18.8% methylmethacrylate.
4. 18.7% n-butylacrylate.
5. 18.8% ethyleneglycoldiacrylate.
6. 18.7% 1,6-hexanediolacrylate.
7. 14.42% dehydrated castor oil + 4.41% vinyl toluene.
8. 20.06% diallylphthalate.
9. 7.9% vinyl toluene + 7.11% triallylcyanurate.
10. 7.5% divinylbenzene + 7.5% triallylcyanurate.
11. 15% 1,6-hexandioldiacrylate + 5% triallylcyanurate.
12. 10.9% vinyl toluene + 4% divinylbenzene + 6% styrene.
13. 50% Bisphenol A dicyanate resin (commercially available from Mitsubishi Gas Co, Tokyo, Japan as BT-2000) as component (A) instead of the hydrocarbon cyanate ester + 50% diallylether of bisphenol A as component (B).

TABLE I

| Sample No. | React. Dil. | Catalyst | | | Accelerator | | Gel Time Sec. | Cure Time Sec. | Peak Exo-therm °C |
|---|---|---|---|---|---|---|---|---|---|
| | | Cation | Anion | ppm | Type | Conc.[b] | | | |
| A | 1 | $Fe^{+3}$ | Naphthenate | 2499 | None | ---- | 161 | 289 | 249.1 |
| B | 1 | $Zn^{+2}$ | Synth. Blend[a] | 2752 | None | --- | 50 | 152 | 227.4 |
| C | 2 | $Fe^{+3}$ | Naphthenate | 2514 | None | ---- | 209 | 361 | 245.8 |
| D | 2 | $Fe^{+2}$ | Octoate | 2514 | None | ---- | 140 | 215 | 220.4 |
| E | 2 | $Fe^{+3}$ | Octoate | 2501 | None | ---- | 121 | 197 | 242.2 |
| F | 1 | $Fe^{+3}$ | Naphthenate | 1201 | None | ---- | 190 | 471 | 229.9 |
| G | 1 | $Fe^{+3}$ | Synth. Blend[a] | 5064 | None | ---- | 75 | 164 | 241.2 |
| H | 1 | $Fe^{+3}$<br>$Zn^{+2}$ | Synth. Blend[a]<br>Synth. Blend[a] | 1258<br>1255 | None | ---- | 97 | 248 | 226.1 |
| I | 3 | $Zn^{+2}$ | Synth. Blend[a] | 2485 | None | ---- | 71 | 278 | 201.9 |
| J | 4 | $Zn^{+2}$ | Synth. Blend[a] | 2574 | None | ---- | 53 | 198 | 206.4 |
| K | 5 | $Zn^{+2}$ | Synth. Blend[a] | 2574 | None | ---- | 70 | 215 | 214.8 |
| L | 6 | $Zn^{+2}$ | Synth. Blend[a] | 2485 | None | ---- | 65 | 218 | 214.6 |

[a] A proprietary mixture of $C_8$-$C_{12}$ aliphatic acids available under the tradename NuXtra™ from Nuodex, Inc. Piscataway, N.J.

[b] % by weight based on total composition.

TABLE I (contd.)

| Sample No. | React. Dil. | Catalyst | | | Accelerator | | Gel Time Sec. | Cure Time Sec. | Peak Exo-therm °C |
|---|---|---|---|---|---|---|---|---|---|
| | | Cation | Anion | ppm | Type | Conc.[b] | | | |
| M | 7 | $Zn^{+2}$ | Synth. Blend[a] | 2486 | None | ---- | 61 | 272 | 140.9 |
| N | 8 | $Zn^{+2}$ | Synth. Blend[a] | 3013 | None | ---- | 67 | 187 | 201.5 |
| O | 9 | $Fe^{+3}$ | Synth. Blend[a] | 2533 | None | ---- | 212 | 309 | 236.2 |
| P | 10 | $Fe^{+3}$ | Synth. Blend[a] | 2531 | None | ---- | 229 | 328 | 248.1 |
| Q | 11 | $Fe^{+3}$ | Synth. Blend[a] | 2533 | None | ---- | 173 | 199 | 270.1 |
| R | 1 | $Fe^{+3}$ | Synth. Blend[a] | 2531 | Phenol | 2.00 | 47 | 124 | 240.3 |
| S | 1 | $Fe^{+3}$ | Synth. Blend[a] | 2533 | Phenol | 1.00 | 76 | 164 | 233.8 |
| T | 12 | $Fe^{+3}$ | Octoate | 2015 | Phenol | 1.00 | 22 | 89 | 206.5 |
| U | 1 | $Fe^{+3}$ | Synth. Blend[a] | 2530 | Hydroquinone | 0.059 | 120 | 237 | 241.9 |
| V | 1 | $Fe^{+3}$ | Synth. Blend[a] | 2511 | Hydroquinone | 1.068 | 78 | 161 | 245.3 |
| W | 2 | $Fe^{+3}$ | Synth. Blend[a] | 2522 | Hydroquinone | 1.005 | 127 | 237 | 237.9 |

[a] A proprietary mixture of $C_8$-$C_{12}$ aliphatic acids available under the tradename NuXtra™ from Nuodex, Inc. Piscataway, N.J.

[b] % by weight based on total composition.

TABLE I (contd.)

| Sample No. | React. Dil. | Catalyst | | | Accelerator | | Gel Time Sec. | Cure Time Sec. | Peak Exo-therm °C |
|---|---|---|---|---|---|---|---|---|---|
| | | Cation | Anion | ppm | Type | Conc. | | | |
| X | 2 | $Fe^{+3}$ | Synth. Blend[a] | 2582 | Hydroquinone | 0.922 | 481[c] | 824[c] | 205.4[c] |
| Y | 2 | $Fe^{+3}$ | Synth. Blend[a] | 2536 | Diallyl bisphenol | 2.87 | 100 | 197 | 239.3 |
| Z | 2 | $Fe^{+3}$ | Synth. Blend[a] | 2563 | 2,6-diallyl phenol | 3.17 | 89 | 173 | 251.1 |
| AA | 13 | $Fe^{+3}$ | Octoate | 2022 | Phenol | 1.03 | 18 | 71 | 205.1 |
| AB | 2 | $Zn^{+2}$ | Synth. Blend[a] | 2490 | Phenol | 1.00 | 34.4 | 144 | 210 |
| AC | 2 | $Fe^{+3}$ | Synth. Blend[a] | 2600 | n-Propanol | 1.00 | 67 | 169 | 242 |
| AD | 2 | $Fe^{+3}$ | ACAC[d] | 2500 | None | ----- | 157 | 532 | 210.7 |
| AE | 2 | $Fe^{+3}$ | ACAC[d] | 2500 | Phenol | 1.00 | 81 | 168 | 206.3 |
| AF | 2 | $Fe^{+3}$ | Synth. Blend[a] | 2509 | None | ----- | 182 | 331 | 247.7 |

[a] A proprietary mixture of $C_8$-$C_{12}$ aliphatic acids available under the tradename NuXtra™ from Nuodex, Inc. Piscataway, N.J.
[b] % by weight based on total composition.
[c] SPI gel/cure test is performed at 65°C to further demonstrate low-temperature cure.
[d] Acetylacetonate

**EXAMPLE 2**

Mixtures of the catalyzed resins in the same proportions as in Example 1 are poured into small pans and cured for times up to 24 hours at 80 °C. The residual amount of cure energy left in the cured resin is measured by differential scanning calorimetry (DSC). The fractional amount of initial cure energy less residual energy over the initial energy is expressed as percent cure and is given in Table II.

TABLE II

| Sample No. | Catalyst | | Percent Cure at 80 °C after | | |
|---|---|---|---|---|---|
| | Cation | Anion | 4 Hours | 8 Hours | 24 Hours |
| A | $Fe^{+++}$ | Naphthenate | 95 | 98 | 95 |
| B | $Zn^{++}$ | Naphthenate | 93 | 93 | 90 |

**EXAMPLE 3**

Into 575 g of 80% by weight of the cyanate ester resin described in Example 1 in vinyl toluene, are added 25 g of iron ($Fe^{+++}$) naphthenate catalyst solution. After thoroughly mixing, the sample is placed inside a vacuum chamber and degassed, divided into two portions and poured into each of two 0.125" (3.175 mm) thick aluminum molds which are then placed in an oven at 80 °C. The castings are cured for 4 hours. One of the castings is further post-cured at 171 °C for 2 hours. The non-postcured casting is 85% cured by DSC whereas the post-cured casting is 100% cured.

After cooling, the cured resins are prepared for testing by the following methods: ASTM D-638 (tensile); ASTM D-790 (flexural) and ASTM D-648 (heat distortion temperature, HDT).

Water absorption is determined from specimens of 1 in. x 3 in. (25.4 mm x 76.2 mm) coupons of the above mentioned castings. The edges of the coupons are sanded successively with 320, 400 and 600 grit emery cloth. The specimens are dried at 90 °C overnight and cooled for 2 hours in a dessicator. The samples are weighed and then immersed in boiling water. The samples are removed, dried and weighed at intervals during the test. The final weighing is performed after 168 hours.

Barcol Hardness is determined from a Barber-Coleman Impressor model GTZJ 934-1 and is expressed as the average of ten measured values.

The glass transition temperature (Tg) is determined by thermomechanical analysis (TMA) on a DuPont instrument model 1090 series 4 thermal analysis instrument.

After the four hour period, the castings are removed from the oven and glass transition temperatures are measured by differential scanning calorimetry (DSC) and thermomechanical analysis (TMA). The residual energy of curing is also measured by DSC.

The results are given in Table III.

**EXAMPLE 4**

Into 590.62 g of 80% by weight of the cyanate ester resin described in Example 1 in vinyl toluene, are added 9.38 g of zinc ($Zn^{++}$) mixed carboxylate catalyst solution. After thoroughly mixing, the sample is placed inside a vacuum chamber and degassed, divided into two portions and poured into each of two 0.125" (3.175 mm) thick aluminum molds which are then placed in an oven at 80 °C. The castings are cured for 4 hours. One of the castings is further post-cured at 171 °C for 2 hours. The non-postcured casting is 85% cured by DSC whereas the post-cured casting is 100% cured. The physical properties of these castings are determined as in Example 3 and the results are given in Table III.

**EXAMPLE 5**

Into 5.77 g of neat ferric octoate (10.4% iron by weight), 3.05 g of 1,6-hexanedioldiacrylate is mixed in to form a thin paste into which 288.53 g of a solution warmed to 55 °C consisting of 85 percent by weight of the 3.2 functioanl polyaryl aliphatic cyanate employed in Example 1, 7.5 weight percent divinylbenzene, 7.15 weight percent triallylcyanurate, and 0.35 weight percent 1,6-hexanedioldiacrylate is thoroughly mixed in. Into the above mixture, 3.01 g of liquid phenol (warmed to 55 °C is added. The solution is thoroughly mixed, followed by degassing. The mixture is then poured into a heated mold (55 °C) consisting of two 0.125 in. (3.175 mm) thick aluminum plates spaced 0.125 in. (3.175 mm) apart. The casting is heated for

251 minutes at 90° C after which the mold is cooled to room temperature and demolded. The physical properties of this casting are determined as in Example 3 and the results are given in Table III.

TABLE III

| | Sample A Ex.(3) | Sample B Ex.(3) | Sample C Ex (4) | Sample D Ex.(4) | Sample E Ex.(5) |
|---|---|---|---|---|---|
| Metal | $Fe^{+++}$ | $Fe^{+++}$ | $Zn^{++}$ | $Zn^{++}$ | $Fe^{+++}$ |
| Conc.,ppm[a] | 2500 | 2500 | 2500 | 2500 | 2000 |
| Cure Sched, hours/° C | 4/80 | 4/80 2/171 | 4/80 | 4/80 2/177 | 4.1/90 |
| T.Strength, | | | | | |
| kpsi | 5.49 | 7.05 | 6.14 | 8.94 | 4.82 |
| ,MPa | 37.9 | 48.6 | 42.3 | 61.6 | 33.2 |
| T.Modulus, | | | | | |
| kpsi | 288.3 | 475.6 | 282.6 | 515.9 | 426.2 |
| ,MPa | 1.99 | 3.28 | 1.95 | 3.56 | 2.94 |
| Elongation, % | 1.49 | 0.8 | 1.87 | 2.06 | 1.35 |
| F.Strength, | | | | | |
| kpsi | 9.41 | 14.42 | 9.46 | 20.86 | 9.41 |
| ,MPa | 64.9 | 99.4 | 65.2 | 143.8 | 64.9 |
| F.Modulus, | | | | | |
| kpsi | 265 | 460.5 | 277.1 | 462.6 | 455.5 |
| ,MPa | 1.83 | 3.18 | 1.91 | 3.19 | 3.14 |
| Barcol Hardness | ------ | 46 | ------ | 46 | 33 |
| Water Abs. % | 3.18 | 1.757 | >9[b] | 2.118 | 4.36 |
| HDTUL, ° C | 61.1 | 156 | 60 | 154 | 99.4 |
| Tg (Dry) | 85.7 | 211.4 | 82.1 | 217 | 111-117 |

[a] Concentration of metal based on total weight of the composition.
[b] Some of the sample decomposed.

## Claims

1. A curable composition comprising (A) at least one polyarylcyanate ester composition; (B) at least one ethylenically unsaturated monomer; (C) as a catalyst for curing, at least one iron or zinc complex; and optionally (D) a hydrogen-bonding compound as a curing accelerator; and wherein component (A) is employed in an amount of from about 50 to about 95 percent by weight based upon the combined weight of components (A) and (B); component (B) is employed in an amount of from about 5 to about 50 percent by weight based upon the combined weight of components (A) and (B); component (C) is employed in an amount of from about 0.01 to about 2 percent by weight based upon the combined weight of components (A), (B), (C) and (D) and component (D) is employed in an amount of from zero to about 10 percent by weight based upon the combined weight of components (A), (B), (C) and (D).

2. A composition of Claim 1 wherein

(a) component (A) is present in an amount of from about 70 to about 95 percent by weight based upon the combined weight of components (A) and (B);

(b) component (B) is present in an amount of from about 5 to about 25 percent by weight based upon the combined weight of components (A) and (B);

(c) component (C) is present in an amount of from about 0.05 to about 1 percent by weight based upon the combined weight of components (A), (B), (C) and (D); and

(d) component (D) is present in an amount of from about 0.05 to about 2 percent by weight based upon the combined weight of components (A), (B), (C) and (D).

3. A composition of Claim 1 wherein

(a) component (A) is present in an amount of from about 75 to about 90 percent by weight based upon the combined weight of components (A) and (B);

(b) component (B) is present in an amount of from about 10 to about 25 percent by weight based upon the combined weight of components (A) and (B);

(c) component (C) is present in an amount of from about 0.1 to about 0.75 percent by weight based upon the combined weight of components (A), (B), (C) and (D); and

(d) component (D) is present in an amount of from about 0.5 to about 1.2 percent by weight based upon the combined weight of components (A), (B), (C) and (D).

4. A composition of Claim 1 wherein the catalyst, component (C), is a ferrous or ferric salt of a monocarboxylic acid having from about 6 to about 12 carbon atoms.

5. A composition of Claim 4 wherein said ferrous or ferric salt of a monocarboxylic acid is ferrous octoate, ferric octoate, ferric naphthenate, a blend of ferric salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

6. A composition of Claim 2 wherein the catalyst, component (C), is a ferrous or ferric salt of a monocarboxylic acid having from about 6 to about 12 carbon atoms.

7. A composition of Claim 6 wherein said ferrous or ferric salt of a monocarboxylic acid is ferrous octoate, ferric octoate, ferric naphthenate, a blend of ferric salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

8. A composition of Claim 3 wherein the catalyst, component (C), is a ferrous or ferric salt of a monocarboxylic acid having from about 6 to about 12 carbon atoms.

9. A composition of Claim 8 wherein said ferrous or ferric salt of a monocarboxylic acid is ferrous octoate, ferric octoate, ferric naphthenate, a blend of ferric salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

10 A composition of Claim 1 wherein the catalyst, component (C), is ferric acetylacetonate.

11. A composition of Claim 2 wherein the catalyst, component (C), is ferric acetylacetonate.

12. A composition of Claim 3 wherein the catalyst, component (C), is ferric acetylacetonate.

13. A composition of Claim 1 wherein the catalyst, component (C), is a zinc salt of a monocarboxylic acid having from about 6 to about 12 carbon atoms.

14. A composition of Claim 13 wherein said zinc salt of a monocarboxylic acid is a blend of zinc salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

15. A composition of Claim 2 wherein the catalyst, component (C), is a zinc salt of a monocarboxylic acid having from about 6 to about 12 carbon atoms.

16. A composition of Claim 15 wherein said zinc salt of a monocarboxylic acid is a blend of zinc salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6- hexanedioldiacrylate, diallylphthalate, diallyl ether of bisphenol A, or a combination thereof.

17. A composition of Claim 3 wherein the catalyst, component (C), is a zinc salt of a monocarboxylic acid having from about 6 to about 12 carbon atoms.

18. A composition of Claim 17 wherein said zinc salt of a monocarboxylic acid is a blend of zinc salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

19. A composition of Claim 1, 2, 3, 4, 5, 6, 7, 8, 9, 10, 11, 12, 13, 14, 15, 16, 17 or 18 wherein

(a) component (A) is a polyarylcyanate ester represented by the following formula

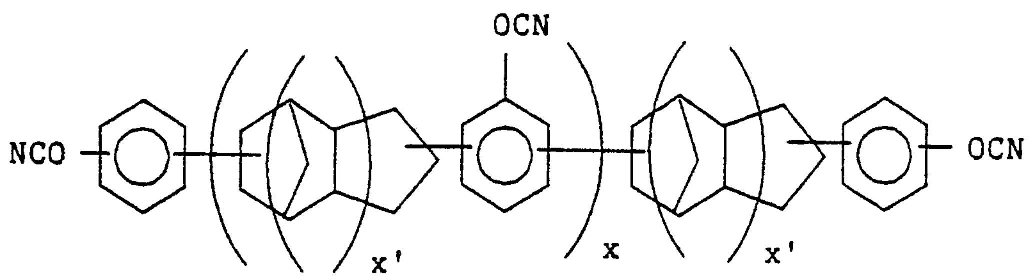

wherein x has an average value from zero up to about 5, inclusive and each x′ independently has an average value from 1 to about 3;

(b) component (B) is styrene, vinyltoluene, divinylbenzene, triallylcyanurate, methylmethacrylate, n-butylacrylate, 1,6- hexanedioldiacrylate, diallylphthalate, diallyl ether of bisphenol A, or a combination thereof; and

(c) component (D), when present, is phenol, hydroquinone, n-propanol, diallylbisphenol A, 2-allylphenol, 2,6-diallylphenol, or a combination thereof.

20. In a method for curing, by heating, a composition comprising (A) at least one polyarylcyanate ester composition; (B) at least one ethylenically unsaturated monomer; (C) a catalyst; and optionally (D) a hydrogen-bonding compound as a curing accelerator; the improvement which comprises employing as the catalyst, component (C), at least one selected from the group consisting of iron and zinc salts of a monocarboxylic acid in an amount of from about 0.01 to about 2 percent by weight based upon the combined weight of components (A) and (B); and wherein the curing is conducted at a temperature of not greater than 100°C.

21. A method of Claim 20 wherein

(a) component (A) is present in an amount of from about 70 to about 95 percent by weight based upon the combined weight of components (A) and (B);

(b) component (B) is present in an amount of from about 5 to about 25 percent by weight based upon the combined weight of components (A) and (B);

(c) component (C) is present in an amount of from about 0.05 to about 1 percent by weight based upon the combined weight of components (A), (B), (C) and (D); and

(d) component (D) is present in an amount of from about 0.05 to about 2 percent by weight based upon the combined weight of components (A), (B), (C) and (D).

22. A method of Claim 20 wherein

(a) component (A) is present in an amount of from about 75 to about 90 percent by weight based upon the combined weight of components (A) and (B);

(b) component (B) is present in an amount of from about 10 to about 25 percent by weight based upon the combined weight of components (A) and (B);

(c) component (C) is present in an amount of from about 0.1 to about 0.75 percent by weight based upon the combined weight of components (A), (B), (C) and (D); and

(d) component (D) is present in an amount of from about 0.5 to about 1.2 percent by weight based upon the combined weight of components (A), (B), (C) and (D).

23. A method of Claim 20 wherein the catalyst, component (C), is a ferrous or ferric salt of a monocarboxylic acid having from about 6 to about 12 carbon atoms.

24. A method of Claim 23 wherein said ferrous or ferric salt of a monocarboxylic acid is ferrous octoate, ferric octoate, ferric naphthenate, a blend of ferric salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

25. A method of Claim 21 wherein the catalyst, component (C), is a ferrous or ferric salt of a monocarboxylic acid having from about 6 to about 12 carbon atoms.

26. A method of Claim 25 wherein said ferrous or ferric salt of a monocarboxylic acid is ferrous octoate, ferric octoate, ferric naphthenate, a blend of ferric salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

27. A method of Claim 22 wherein the catalyst, component (C), is a ferrous or ferric salt of a monocarboxylic acid having from about 6 to about 12 carbon atoms.

28. A method of Claim 27 wherein said ferrous or ferric salt of a monocarboxylic acid is ferrous octoate, ferric octoate, ferric naphthenate, a blend of ferric salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

29 A method of Claim 20 wherein the catalyst, component (C), is ferric acetylacetonate.

30. A method of Claim 21 wherein the catalyst, component (C), is ferric acetylacetonate.

31. A method of Claim 22 wherein the catalyst, component (C), is ferric acetylacetonate.

32. A method of Claim 20 wherein the catalyst, component (C), is a zinc salt of a monocarboxylic acid having from about 6 to about 12 carbon atoms.

33. A method of Claim 32 wherein said zinc salt of a monocarboxylic acid is a blend of zinc salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

34. A method of Claim 21 wherein the catalyst, component (C), is a zinc salt of a monocarboxylic acid having from about 6 to about 12 carbon atoms.

35. A method of Claim 34 wherein said zinc salt of a monocarboxylic acid is a blend of zinc salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

36. A method of Claim 22 wherein the catalyst, component (C), is a zinc salt of a monocarboxylic acid having from about 6 to about 12 carbon atoms.

37. A method of Claim 36 wherein said zinc salt of a monocarboxylic acid is a blend of zinc salts of $C_8$-$C_{12}$ aliphatic monocarboxylic acids, and the ethylenically unsaturated monomer (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof.

38. A method of Claim 20, 21, 22, 23, 24, 25, 26, 27, 28, 29, 30, 31, 32, 33, 34, 35, 36 or 37 wherein

(a) component (A) is a polyarylcyanate ester represented by the following formula

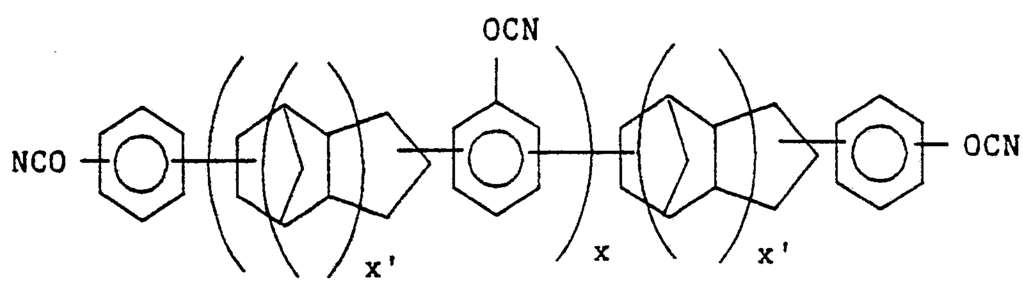

wherein x has an average value from zero up to about 5, inclusive and each x′ independently has an average value from 1 to about 3;

(b) component (B) is styrene, vinyltoluene, divinylbenzene, triallylcyanurate, methylmethacrylate, n-butylacrylate, 1,6-hexanedioldiacrylate, diallylphthalate, diallyl ether of bisphenol A, or a combination thereof; and

(c) component (D), when present, is phenol, hydroquinone, n-propanol, diallylbisphenol A, 2- allylphenol, 2,6-diallylphenol, or a combination thereof.

39. An article resulting from the method of Claim 20, 21, 22, 23, 24, 25, 26, 27, 28, 29, 30, 31, 32, 33, 34, 35, 36, or 37 which product also contains a reinforcing agent.

40. An article resulting from the method of Claim 38 which product also contains a reinforcing agent.

41. An article of Claim 39 wherein said reinforcing agent is glass, aramid, graphite or a combination thereof in woven, mat, roving or random fibrous form.

42. An article of Claim 41 wherein said reinforcing agent is glass, aramid, graphite or a combination thereof in woven, mat, roving or random fibrous form.